Europäisches Patentamt

European Patent Office

Office européen des brevets

(19)

(11) Veröffentlichungsnummer: **0 008 751**
**A1**

(12) # EUROPÄISCHE PATENTANMELDUNG

(21) Anmeldenummer: 79103119.8

(22) Anmeldetag: 24.08.79

(51) Int. Cl.³: **D 04 H 1/46**
**A 61 F 13/20**

(30) Priorität: 05.09.78 DE 2838617

(43) Veröffentlichungstag der Anmeldung:
19.03.80 Patentblatt 80/6

(84) Benannte Vertragsstaaten:
AT CH DE FR GB IT SE

(71) Anmelder: BAYER Aktiengesellschaft
Zentralbereich Patente,Marken und Lizenzen Bayerwerk
D-5090 Leverkusen 1(DE)

(72) Erfinder: Graf, Heinz, Dipl.-Ing.
Eulenweg 2
D-4047 Dormagen(DE)

(72) Erfinder: Dorsch, Peter, Dr.
Bahnhofstrasse 39
D-4047 Dormagen 1(DE)

(72) Erfinder: Lumpp, Kurt
Dieplinghof 3
D-4047 Dormagen(DE)

(54) Verfahren zur Verfestigung von Wattebändern zur Tampon-Herstellung aus Synthesefasern.

(57) Die Erfindung betrifft ein Verfahren zur Herstellung von verfestigten Wattebändern zur Tampon-Herstellung aus Synthesefasern, wobei zuerst ein mehrschichtiges Vlies hergestellt wird, dieses durch leichtes vernadeln verfestigt und dann auf die gewünschte Wattebandbreite zerschnitten wird.

EP 0 008 751 A1

0008751

BAYER AKTIENGESELLSCHAFT          5090 Leverkusen, Bayerwerk

Zentralbereich                   Dn/bc
Patente, Marken und Lizenzen

Verfahren zur Verfestigung von Wattebändern zur Tampon-
Herstellung aus Synthesefasern

Bei der Herstellung von Tampons für die Frauen-Hygiene
werden Wattebänder auf der Tampon-Maschine verarbeitet.
Um die übliche Lagerhaltung dieser Wattebänder und ihre
störungsfreie Verarbeitung auf der Tampon-Maschine zu
ermöglichen, müssen die Wattebänder zuvor verfestigt
werden.

Die auf diesem Einsatzgebiet üblicherweise verwendeten
Wattebänder aus Baumwollfasern oder Regenerat-Zellulosefasern lassen sich auf einfache Weise durch eine Kalanderpressung verfestigen. Dadurch, daß diese Fasern im
Vergleich zu Synthesefasern eine geringere Kräuselung,
geringere Biegesteifigkeit, geringere Bauschelastizität
und eine andersartige Oberflächenbeschaffenheit aufweisen, gelingt diese Verfestigung leicht und formbeständig.

Le A 19 105

Setzt man jedoch statt der Baumwollfasern Synthesefasern ein, so lassen sich durch ein einfaches mechanisches Verpressen keine zufriedenstellenden Bandfestigkeiten erzielen, da die Bänder wieder aufspringen. Daher ist ein rationelles Verarbeiten auf der Tampon-Maschine nicht möglich.

Damit läßt sich das beim Einsatz von zellulosischen Fasern durchgeführte Verfahren mit den Verfahrensschritten:

Krempelvliesherstellung - Vliesverdichtung zum Watteband - Kalanderpressung - Bandablage nicht auf synthetische Fasern anwenden.

Es wurde nun gefunden, daß trotzdem synthetische Fasern zu einem für die Verarbeitung der Tamponmaschine brauchbaren Watteband verarbeitet werden können, wenn man das Vlies zuvor leicht vernadelt. Daraus ergibt sich das folgende einfache Produktionsschema:

Flockeauflösung - Erstellung eines mehrschichtigen Vlieses - leichtes Vernadeln zur Verfestigung - Schneiden des Nadelvlieses auf die gewünschte Wattebandbreite - Bandablage.

Die Erfindung betrifft daher ein Verfahren zur Herstellung von verfestigten Wattebändern zur Tampon-Herstellung aus Synthesefasern, das dadurch gekennzeichnet ist, daß zuerst ein mehrschichtiges Vlies hergestellt wird und dieses durch leichtes Vernadeln verfestigt und dann auf die gewünschte Wattebandbreite zerschnitten wird.

Bei diesem Verfahren werden vorzugsweise solche Synthesefasern eingesetzt, die im Hinblick auf das Einsatzgebiet eine ausreichende Flüssigkeitsspeicherung und Saugfähigkeit haben. Unter diesen sind besonders die in der DE-OS
2 554 154 beschriebenen Fasern, insbesondere die auf Acrylnitrilbasis, zu nennen.

Le A 19 105

- 4 -

Patentansprüche

1) Verfahren zur Herstellung von verfestigten Wattebändern zur Tampon-Herstellung aus Synthesefasern, dadurch gekennzeichnet, daß zuerst ein mehrschichtiges Vlies hergestellt wird und dieses durch leichtes Vernadeln verfestigt und dann auf die gewünschte Wattebandbreite zerschnitten wird.

2) Verfahren nach Anspruch 1, dadurch gekennzeichnet, daß die Synthesefasern eine ausreichende Flüssigkeitsspeicherung und Saugfähigkeit haben.

Le A 19 105

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>FR - A - 1 495 510</u> (ROUBANE)<br><br>* Spalte 1, Zeilen 25-28; Zu-<br>sammenfassung 1°,2°,3° * <br><br>---- | 1,2 |

**KLASSIFIKATION DER ANMELDUNG (Int.Cl.⁴)**

D 04 H   1/26
A 61 F   13/20

**RECHERCHIERTE SACHGEBIETE (Int. Cl.³)**

D 04 H   1/46
A 61 F   13/20

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-12-1979 | ELSEN-DROUOT |

EPA form 1503.1   06.78